# EUROPEAN PATENT SPECIFICATION

(11) **EP 1 329 189 B1**
(45) Date of publication and mention of the grant of the patent: **04.11.2009**
(21) Application number: 02026401.6
(22) Date of filing: 26.11.2002
(51) Int. Cl.: A61B 1/05, G02B 27/46

(54) **Endoscopic capsule**
Endoskopische Kapsel
Capsule endoscopique

(30) Priority: 18.01.2002 JP 2002010006
(43) Date of publication of application: 23.07.2003
(73) Proprietor: Olympus Corporation, Tokyo (JP)
(72) Inventor: Matsumoto, Shinya, Machida-shi, Tokyo (JP)
(74) Representative: von Hellfeld, Axel

(56) References cited:
- US-A- 5 748 371
- US-A1- 2001 035 902
- US-B1- 6 241 656
- PATENT ABSTRACTS OF JAPAN vol. 2000, no. 21, 3 August 2001 (2001-08-03) & JP 2001 091860 A (ASAHI OPTICAL CO LTD), 6 April 2001 (2001-04-06)

## Description

### BACKGROUND OF THE INVENTION

Conventional endoscopes are formed of a first part with a tip portion that is inserted into a patient's body for observation and treatment, and a control unit that is externally provided and connected to the first part. The first part has electrical devices such as an illumination system and an image sensor within the tip portion. The control unit powers these electrical devices via electric wires.

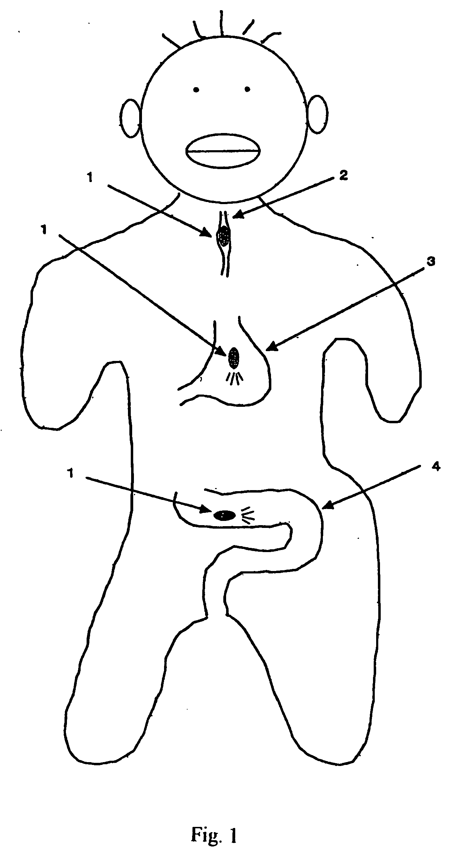

Conventional endoscopes cause significant pain to the patient when they are inserted into the body. For instance, the patient suffers from significant pain when the tip portion passes through the patient's throat. Moreover, the patient continuously suffers from pain while the tip portion is in the body, thus creating a burden to the patient. To reduce the patient's pain, a small, capsule endoscope body 1 as illustrated in Fig. 1 has been proposed. The patient simply swallows the capsule endoscope body 1 and generally experiences little or no pain thereafter during the time that the capsule endoscope body 1 is within the patient's body.

However, the capsule endoscope body 1 has the following problems.

First, it includes batteries which supply the power necessary for making observations within the patient's body; however, the supply of energy is limited. Usually, approximately 30 hours elapses from the time a capsule endoscope body 1 is swallowed until it is discharged from the body. Because of a shortage of power, conventional capsule endoscopes do not allow observations of the entire path within the body. In order to solve this problem, using more batteries or a battery with increased storage capacity has been considered. However, an increase in battery volume causes the required size of the capsule to increase.

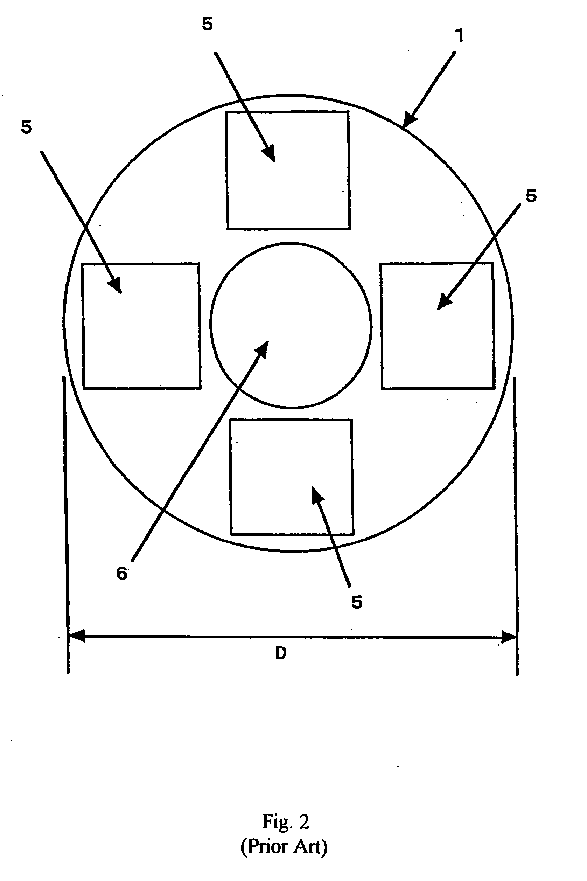

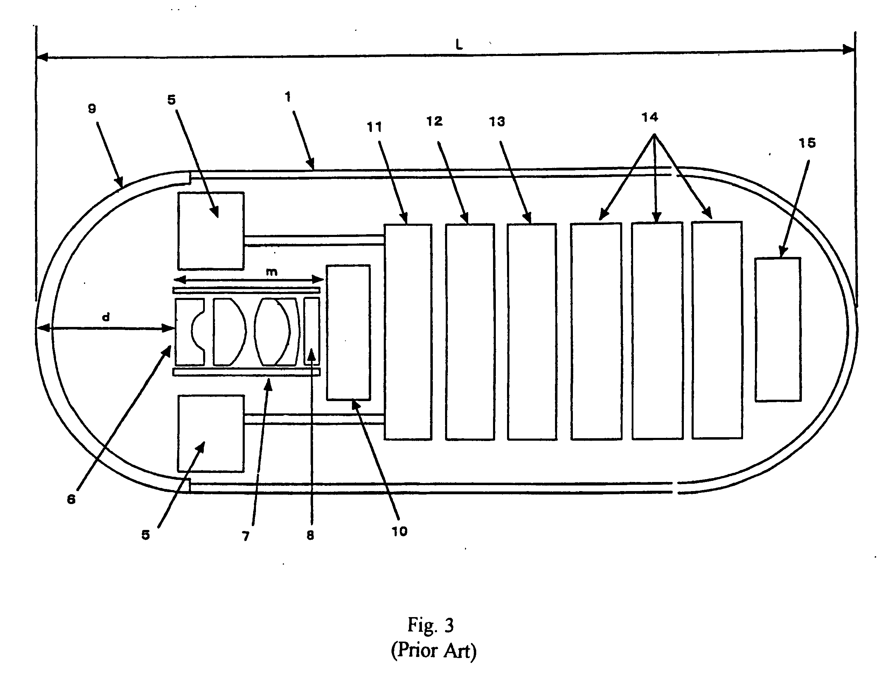

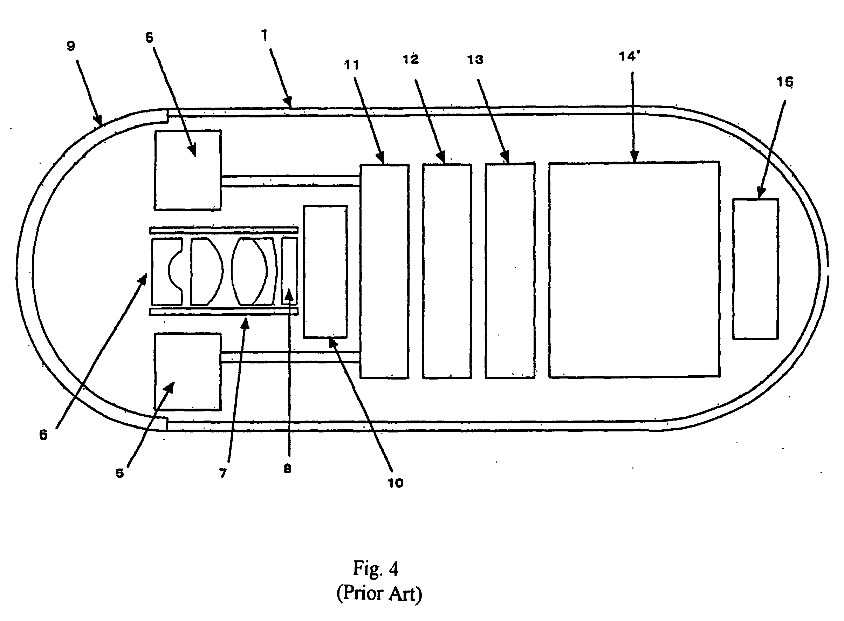

Second, illumination sources as used with conventional capsule endoscopes include halogen lamps and LEDs which provide sufficient brightness for observation in narrow, tubular parts of the body such as the esophagus 2, but yield insufficient illumination for observation within larger spaces, such as the stomach 3 and the large intestine 4. To overcome this, larger LEDs 5, such as shown in Fig. 2, are used to ensure a sufficient brightness. However, the diameter D of the capsule must be increased with an increase in the size of the illumination system. In addition, operating larger LEDs requires more power. To overcome this, either more batteries 14 (Fig. 3) or a battery 14' (Fig. 4) with increased storage capacity are generally needed. As a result, the overall length L of the capsule must be increased in order to accommodate the necessary increase in volume of the battery or batteries. However, an increase in overall length of the capsule impairs the capsule endoscopes advantage of reducing the patient's pain.

One of the requirements for capsule endoscopes is that the objective lens 6 (Fig. 2) has a large depth of field that spans from the exterior surface of the tip portion cover 9 (Fig. 3) to several tens of millimeters in front of it. Generally, an objective optical system with a higher F number will have a greater depth of field. However, using an objective optical system that has a higher F number will generally restrict the light rays passing through the objective optical system, thereby making the image less bright. To counteract this, the illumination source must be made brighter.

Capsule endoscopes carry a relatively weak illumination source, as described above. Therefore, when the objective optical system has a large F number, the image can be so dark that observation and diagnosis of an object become difficult to perform. For this reason, conventional capsule endoscopes do not extend the depth of field of the objective optical system by using an objective optical system having a high F number. Consequently, conventional capsule endoscopes have a small depth of field.

In order to meet the viewing requirements, the objective optical system 7 of a conventional capsule endoscope, as shown in Fig. 3, is designed to focus at a surface several tens of millimeters away from the front end of the objective optical system. The distance d between the tip portion cover 9 and the front end of the objective optical system is adjusted in accordance with the depth of field of the objective optical system so that the near point of the depth of field matches the exterior surface of the tip portion cover 9. This allows an object to be in focus from the tip portion cover to several tens of millimeters away from the tip portion cover. However, the configuration as shown in Fig. 3 increases the distance d between the tip portion cover and the front end of the objective optical system, and thus results in an increase in overall capsule length L.

The objective optical system of a capsule endoscope is also required to be small. The objective optical system in conventional, non-capsule endoscopes is formed of, for example, many lenses and various filters, the latter being for color correction. For a conventional capsule endoscope, color correction ensures a constant color reproduction when used in combination with an illumination system that employs sources having different spectral outputs. Solid-state elements'such as CCD and CMOS devices are especially sensitive to infrared wavelengths. Thus, this nonlinear sensitivity can result in there being optical noise that is introduced during the imaging process. Therefore, a filter to reduce the intensity of infrared wavelengths is generally positioned within the objective optical system. For this reason, the objective optical system of a conventional, non-capsule endoscope has a large overall length. Because it is formed of many optical elements, the objective optical system of a conventional, non-capsule endoscope suffers from both high cost of components and high cost of assembly. Thus, prior art objective optical systems for non-capsule endoscopes are not appropriate for use as an objective optical system for a capsule endoscope.

A capsule endoscope body may be controlled by magnetic induction in order to affect its location/orientation during the observation period within a patient's body. Therefore, it is important that the capsule endoscope body be lightweight. The capsule endoscope body is also required to be disposable. Therefore, reducing the production cost per capsule is crucial.

To satisfy the above requirements, the objective optical system in capsule endoscopes may be formed using plastic lenses. However, plastic lenses are subject to relatively large changes in their shape, depending on the temperature and amount of hydration of the plastic lenses. Changes in physical characteristics, such as the refractive index, also occur. Thus, the temperature, amount of moisture in the body, and elapsed time since being ingested, greatly affect the imaging performance of the objective optical system. This leads to a problem in that changes in the depth of field of the objective optical system that occur during the course of an observation within a patient's body may result in a failure of the capsule endoscope to provide needed images. To avoid this, the prior art has considered variations in the production tolerances of the objective optical system and variations in the depth of field under actual circumstances, so that focusing is performed with higher precision. This makes assembly more difficult, decreases the yield of acceptable product, and thus results in an increase in the cost of production.

Document US 2001/0035902 A1 concerns a swallowable capsule having an optical window and containing an imaging system for obtaining in vivo images of the entire length of a gastrointestinal tract and an transmitter which transmits the obtained images to a receiving system.

Document US 5,748,371 concerns an apparatus for increasing the depth of field of an incoherent optical system having an optical transfer function comprising an optical mask being constructed and arranged to alter the optical transfer function such that it is substantially insensitive to an unknown distance between an object and a lens over a greater range of object distances than was provided by the unaltered optical transfer function.

Document US 6,241,656 B1 concerns an endoscope system to which a plurality of different types of endoscopes can be selectively connected. At least one of the endoscopes has a spatial frequency characteristic converter, such as a pupil modulation element, provided in an imaging optical system.

### BRIEF SUMMARY OF THE INVENTION

A first object of the invention is to ensure a large depth of field for a capsule endoscope; a second object of the invention is to extend the time period that observations may be taken using a capsule endoscope; a third object of the invention is to make a capsule endoscope body smaller in size; a fourth object of the invention is to reduce the costs of components within a capsule endoscope body; and a fifth object of the invention is to reduce the costs of assembly of a capsule endoscope body.
The invention is also concerned with a system comprising in combination a capsule endoscope body and a capsule endoscope receiver for receiving image data transmitted from the capsule endoscope body.

The solutions to the above-stated technical problems in accordance with the present invention, are characterized in independent claim 1. Preferred embodiments of the invention are described in the dependent claims.

### BRIEF DESCRIPTION OF THE DRAWINGS

The present invention will become more fully understood from the detailed description given below and the accompanying drawings, which are given by way of illustration only and thus are not limitative of the present invention, wherein:
Fig. 1 is a schematic diagram of portions of the interior of a human body relevant to the present invention;
Fig. 2 is a schematic diagram showing a front view of a capsule endoscope body of the prior art;
Fig. 3 is a schematic diagram that shows the internal structure of the capsule endoscope body shown in Fig. 2;
Fig. 4 is a schematic diagram that shows the internal structure of another capsule endoscope body of the prior art;
Fig. 5 shows the overall configuration of a capsule endoscope observation system according to the present invention;
Fig. 6 is a schematic diagram that shows the internal structure of a capsule endoscope body according to Embodiment 1 of the present invention;
Fig. 7 is a schematic diagram showing a front view of the capsule endoscope boc according to Embodiment 1 of the present invention;
Fig. 8 is a schematic diagram that shows the internal structure of a capsule endo/scope body according to Embodiment 2 of the present invention;
Fig. 9 is a schematic diagram that shows the internal structure of a capsule endo/scope body according to Embodiment 3 of the present invention;
Fig. 10 is a schematic diagram that shows the internal structure of a capsule endoscope body according to Embodiment 5 of the present invention;
Fig. 11 is a schematic diagram that shows the internal structure of a capsule endoscope body according to Embodiment 6, considered the "best mode", of'the present invention;
Figs. 12(a) and 12(b) show the detailed structure of the objective optical system the capsule endoscope of Embodiment 6, which embodiment is currently the "best mod of the invention, with Fig. 12(a) being a side view and Fig. 12(b) being an end view as seen from the object side;
Fig. 13 shows the detailed structure of another example of an objective optical system that may be used in the capsule endoscope of Embodiment 6;
Fig. 14 shows the internal structure of the capsule endoscope body of Embodiment 7;
Figs. 15(a) and 15(b) show the detailed structure of the objective optical system Embodiment 7, with Fig. 15(a) being a side cross-sectional view, and Fig. 15(b) being a end view, as seen from the object side;
Figs. 16(a) and 16(b) show the detailed structure of another example of the objective optical system of Embodiment 7, with Fig. 16(a) being a side cross-sectional view, and Fig. 16(b) being an end view, as seen from the object side;
Fig. 17 shows the internal structure of the capsule endoscope body of Embodiment 8 of the present invention;
Fig. 18 shows the detailed structure of the objective optical system of Embodiment 8 of the present invention;
Figs. 19(a) and 19(b) show the detailed structure of the objective optical system of Embodiment 9 of the present invention, with Fig. 19(a) being a side cross-sectional view, and Fig. 19(b) being an end view, as seen from the object side;
Fig. 20 is a schematic front view of the capsule endoscope body of Embodiment 10 of the present invention;
Fig. 21 shows the detailed structure of the objective optical system of Embodiment 10 of the present invention;
Fig. 22 shows an extended depth of field imaging system of the prior art, which heretofore has not been used to extend the depth of field of a capsule endoscope imaging system;
Fig. 23 is a perspective view to show the appearance of the mask, shown in Fig. 22, which functions as a spatial frequency characteristic converter to cause the optical transfer function of the imaging system of Fig. 22 to remain essentially constant within some range of in-focus position;
Fig. 24 is a graphical presentation to show the intensity profile of the optical transmission function when an object is at the focal point in a general optical system;
Fig. 25 is a graphical presentation to show the intensity profile of the optical transmission function when an object is away from the focal point in a general optical system;
Fig. 26 is a graphical presentation to show the intensity profile of the optical transmission function when an object is farther away from the focal point than in Fig. 25 in a general optical system;
Fig. 27 is a graphical presentation to show the intensity profile of the optical transmission function when an object is at the focal point in an optical system having an extended depth of field;
Fig. 28 is a graphical presentation to show the intensity profile of the optical transmission function when an object is away from the focal point in an optical system having an extended depth of field;
Fig. 29 is a graphical presentation to show the intensity profile of the optical transmission function when an object is further away from the focal point than in Fig. 28 in an optical system having an extended depth of field;
Fig. 30 is a graphical presentation to show the characteristic of an inverse filter for processing the intensity profile of the optical transmission function in an optical system having an extended depth of field;
Fig. 31 is a graphical presentation to show the intensity profile of the optical transmission function after the intensity profile of the optical transmission function of Fig. 27 is processed using the inverse filter having the characteristic of Fig. 30;
Fig. 32 is a graphical presentation to show the intensity profile of the optical transmission function after the intensity profile of the optical transmission function of Fig. 28 is processed using the inverse filter having the characteristic of Fig. 30;
Fig. 33 is a graphical presentation to show the intensity profile of the optical transmission function after the intensity profile of the optical transmission function of Fig. 29 is processed using the inverse filter having the characteristic of Fig. 30;
Fig. 34 is a graphical presentation to show the spectral radiance property of an LED; and
Fig. 35 shows another embodiment of the invention illustrating a combination of a capsule and a receiver.

### DETAILED DESCRIPTION

The capsule endoscope according to the present invention has the following characteristics. A capsule endoscope body includes an illumination system for illuminating the interior of a living body, an imaging system for imaging the interior part that is illuminated, and a transmitter for transmitting image signals captured and output by the imaging system, all of which are housed in a sealed capsule.

The imaging system is formed of an objective optical system, a spatial frequency characteristic converter, and an image sensor which scans the images so as to convert the images into electrical output signals. The spatial frequency characteristic converter consists of an optical mask as taught in U.S. Patent No. 5,748,371. The spatial frequency characteristic convertor causes the optical transfer function to remain essentially constant within some range of in-focus position. This enables the depth of field of the imaging system to be increased while retaining good image quality.

A signal processor, as also taught in U.S. Patent No. 5,748,371, is used to restore the spatial frequency property that has been transformed by the spatial frequency characteristic converter. A signal processor is provided within the capsule endoscope body itself.

A receiver for receiving image signals that are transmitted from the capsule endoscope body is provided away from the capsule endoscope body. The receiver preferably includes the signal processor which restores the spatial frequency property that has been transformed by the spatial frequency characteristic converter within the capsule endoscope body to thereby produce high-resolution images of the interior part over an extended depth of field. The image sensor is a MOS type image sensor. As in prior art capsule endoscopes, the power source for operating the capsule endoscope may include one or more batteries.

According to the present invention, a power source for at least partially powering the capsule endoscope body may be provided outside of the capsule endoscope body. For example, the capsule endoscope body may be powered in whole or in part by electromagnetic waves, such as microwaves, which are transmitted from an external power source. The objective optical system may be formed of plural plastic lenses. The sealed capsule has a transparent tip portion cover that covers the front of the objective optical system and an illumination system. Furthermore, the tip portion cover may have a substantially oval shape.

Alternatively, the tip portion cover may have a shape at the part that covers the front of the objective optical system that is different from the part that covers the front of the illumination system. Furthermore, a member that provides a shielding effect may be provided between the objective optical system and the illumination system. The spatial frequency characteristic converter preferably has an aperture which is analogous in shape to that of the light receiving part of the image sensor, which is generally rectangular in shape. The lens frame for supporting the objective optical system has an aperture on the object side that also serves as a brightness stop. The shape of the brightness stop is also preferably the same as that of the spatial frequency characteristic converter. The objective optical system may be formed of a single aspherical lens of positive refractive power, a lens group having positive refractive power which consists of two positive lenses, or a first lens group having an overall negative refractive power and a second lens group having an overall positive refractive power.

A lens frame for supporting the objective optical system has an opening on the object side that serves as a brightness stop, with the spatial frequency characteristic converter being positioned at substantially the same position as the brightness stop of the objective optical system.

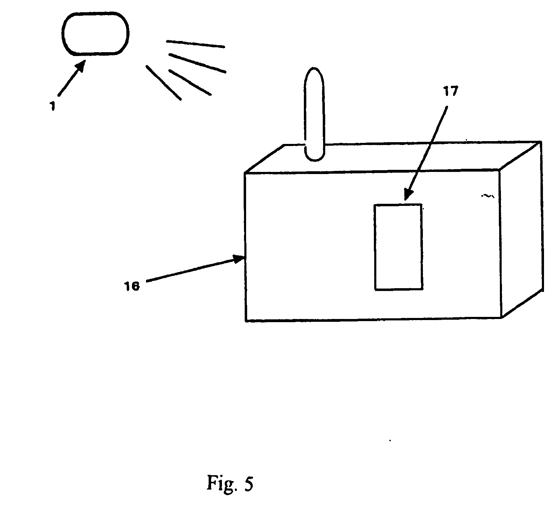

Fig. 5 shows the overall configuration of a capsule endoscope system according to the present invention, comprising a capsule endoscope body 1 and a receiver unit 16 that is set up nearby to receive images from the capsule endoscope body so that the received images may be displayed on a monitor.

### Various embodiments of the invention will now be set forth in detail

### Embodiment 1

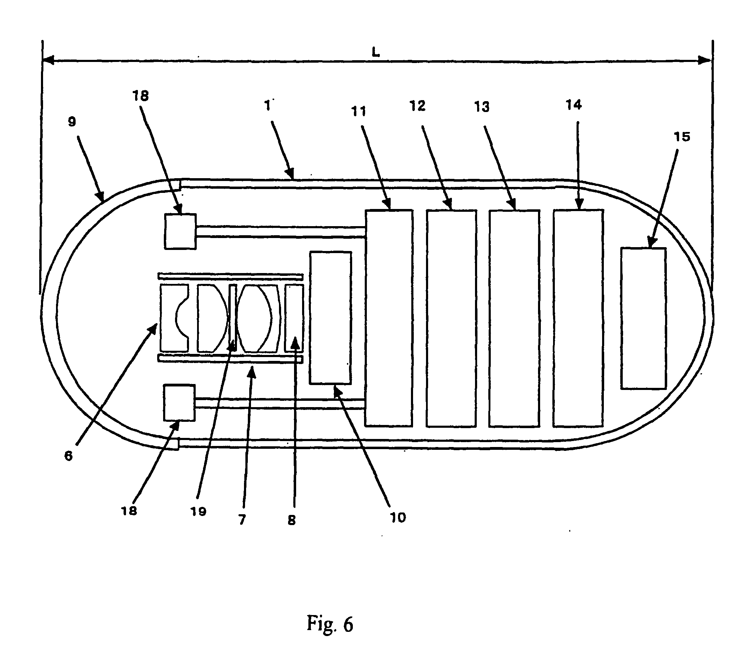

Fig. 6 shows the structure of a capsule endoscope body 1 according to Embodiment 1 of the present invention. The capsule endoscope body is formed of a transparent cover 9 for enclosing the capsule endoscope body 1, an illumination system 18, an objective optical system 7 including an objective lens 6, an infra-red blocking filter 8, a solid-state imaging element 10, an image processing unit 12 for controlling the solid-state imaging element 10 and processing images, a control unit 11, a wireless unit 13, an antenna 15, and a power unit 14. This embodiment uses batteries for the power unit 14.

An objective optical system 7 is provided with a spatial frequency characteristic converter 19 for transforming the spatial frequencies. Also, a signal processing circuit is included in an image processing unit 12 that is provided within the endoscope capsule body. Before shipping of the capsule endoscope body, the image processing unit 12 is used to adjust for variations in optical performance due to production tolerances of the objective optical system 7. This enables the capsule endoscope system to have a constant imaging performance despite differences in optical performance among the individual capsule endoscope bodies, and also improves the production yield by reducing the number of capsule endoscope bodies that must be rejected for quality control reasons. The signal processing circuit is used to restore, using digital processing, the spatial frequency content of image signals so as to remove variations in the spatial content of image signals due merely to production tolerances of optical systems of individual capsule endoscope bodies.

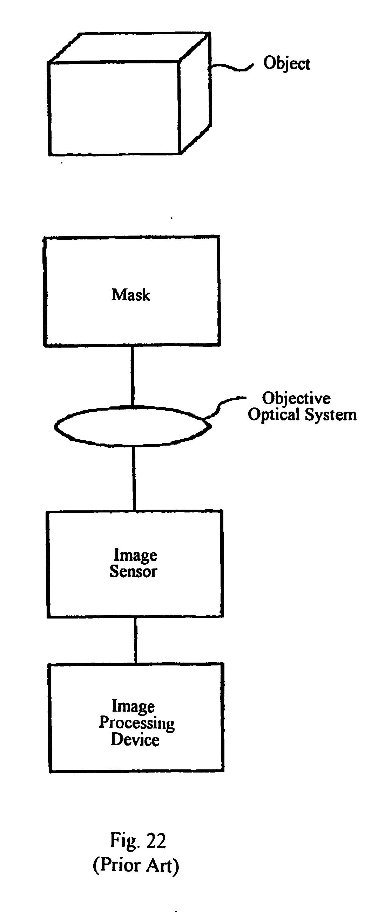

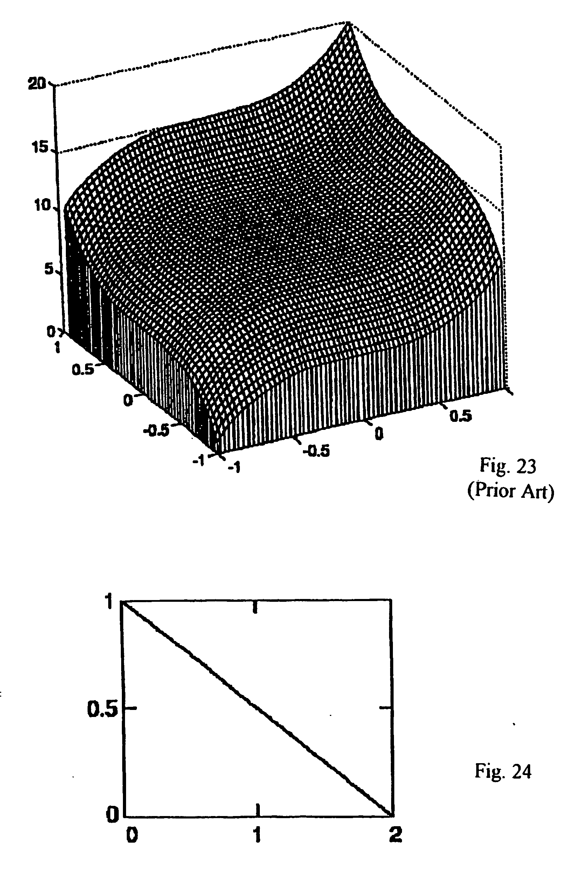

As mentioned above, an extended depth of field optical system is disclosed in U.S. Patent No. 5,748,371. Fig. 22 is a schematic illustration of such a prior art optical system which heretofore has not been used in conjunction with the imaging system of a capsule endoscope. The extended depth of field optical system is formed of an objective optical system (such as the positive lens illustrated) which forms an image of an object by observing the object through a mask. The mask is positioned at the pupil position of the objective optical system, and preferably is a transparent phase mask having a shape as illustrated in Fig. 23. The mask affects the optical transfer function of the optical system by delaying certain spatial frequencies of light from the object more than other spatial frequencies. An image sensor, such as the CCD array illustrated in Fig. 22, captures the modified image data. An image processing device, such as the digital processing system illustrated, is used to undo the effects of the mask, to thereby enable images with an extended depth of field to be displayed for viewing.

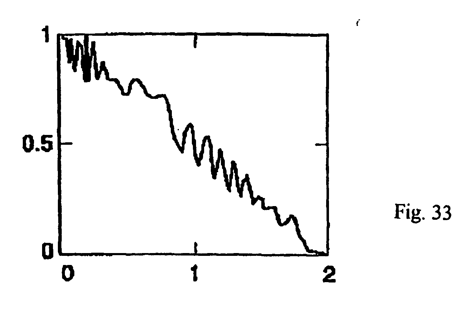

Figs. 24 to 33 are graphs of the optical transmission function OTF (plotted on the Y-axis) versus the relative spatial frequencies at the image plane (plotted on the X-axis), with the number "2" on the X-axis corresponding to the Nyquist frequency for the imaging element.

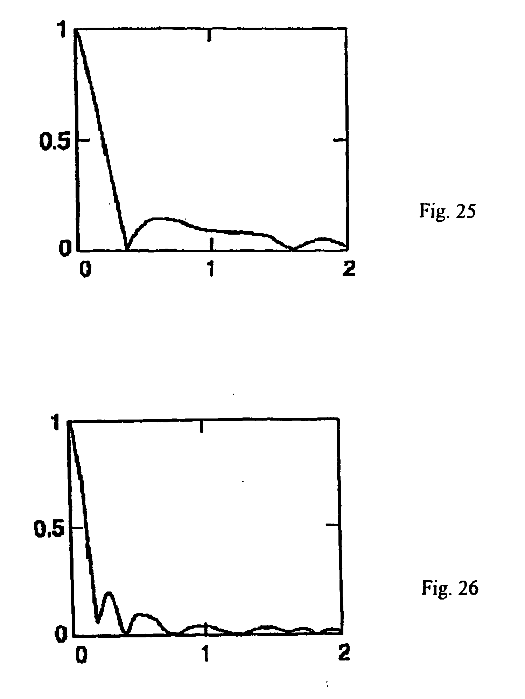

For a conventional optical imaging system without a phase mask; the optical transmission function is as shown in Fig. 24 when an object is positioned at the focal point of the optical system. When the object is moved a given distance away from the focal point of the optical system, the optical transmission function degrades from that shown in Fig. 24 to that shown in Fig. 25. If the object is moved still farther from the focal point of the optical system, the optical transmission function degrades still further as shown in Fig. 26.

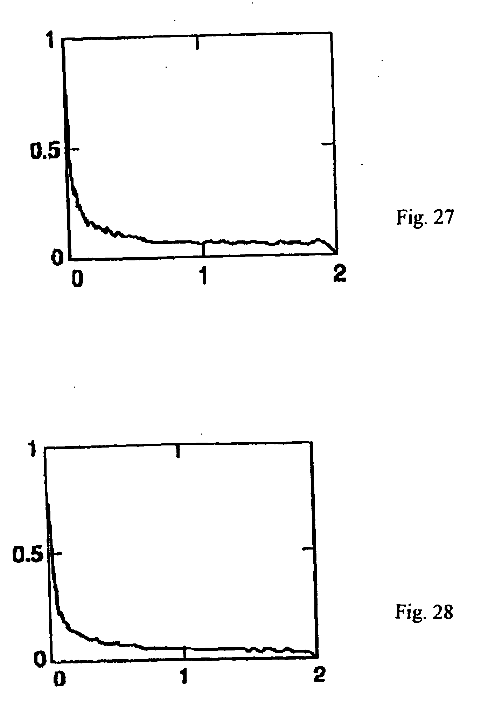

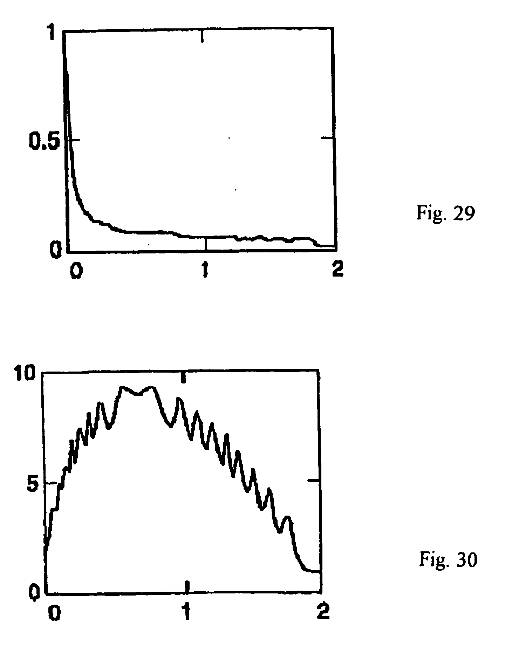

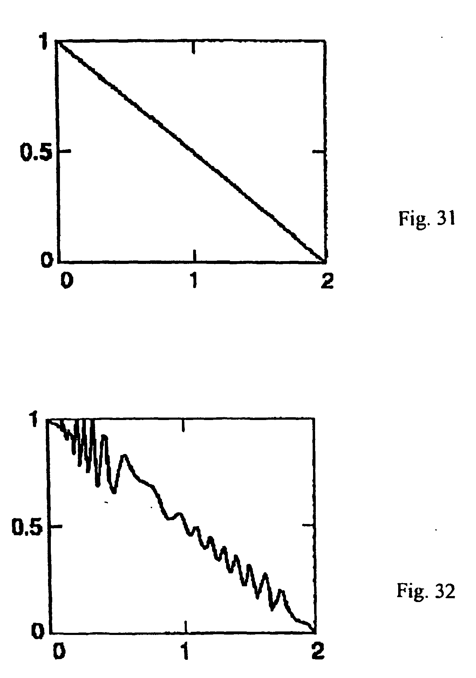

On the other hand, using an optical system having the same optical performance but with an optical phase mask as shown in Fig. 23 (which functions as a spatial frequency characteristic converter) results in the optical transfer functions being as illustrated in Figs. 27, 28, and 29, for the same respective positions of the object relative to the focal point of the optical system. If a filtering process is performed for the intensity profiles shown in Figs. 27, 28, and 29 using an inverse filter having a characteristic as shown in Fig. 30, the OTF profiles become as shown in Figs. 31, 32, and 33, respectively, which are similar to the OTF profiles on the image plane when the object is at the focal point.

As mentioned above, the spatial frequency characteristic converter that is used in the capsule endoscope body of the present invention causes the optical transfer function of the imaging system to remain substantially unchanged within some range of in-focus position, as illustrated by the nearly flat regions of the curves illustrated in Figs. 27, 28 and 29. The spatial frequency characteristic converter 19 may be formed substantially at a pupil of the objective optical system 7, as shown in Fig. 6. Signal processing is then performed to restore the spatial frequency of image signals obtained from a solid-state imaging element 10 that is positioned at the image plane of the optical system. This can overcome the problems with conventional capsule endoscopes and provide an imaging optical system with a greatly increased depth of field.

Thus, the objective optical system 7 can have a small F number and simultaneously provide a large depth of field so as to ensure that bright images can be formed on the solid-state imaging element 10. This allows a capsule endoscope body to obtain images of the interior parts of larger spaces within a living body, such as required when the capsule endoscope is within the stomach and large intestine.

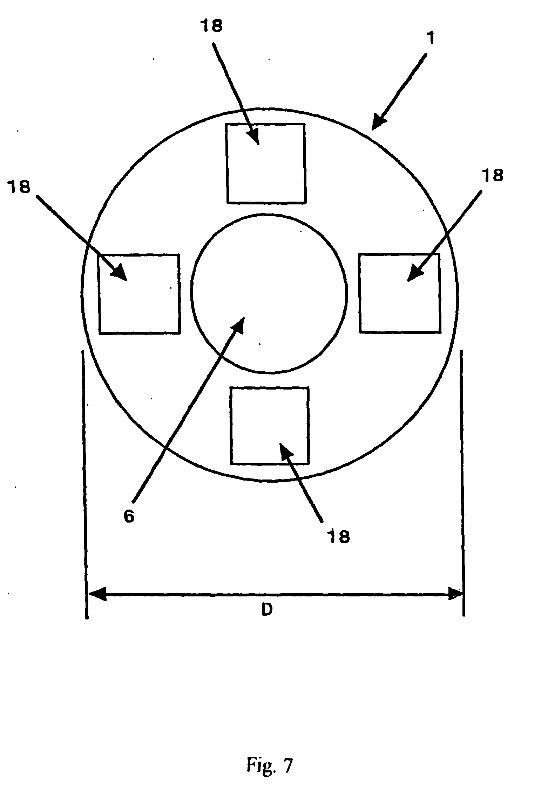

Enabling the objective optical system to have a small F number allows the illumination system 18 to be designed as a low power LED, which leads to downsizing of the illumination system and reducing its power consumption. More advantageously, the capsule endoscope body 1 can now be designed to have a smaller diameter D as is shown in Fig. 7, reducing the patient's pain. Surplus power as a result of using a smaller LED that requires less power allows an extended time for observation and diagnosis by the capsule endoscope body when within a patient. The battery 14 (Fig. 6) can have a significantly reduced capacity and, therefore, a reduced volume. This can shorten the overall length L of the capsule 1 as is shown in Fig. 6.

The extended depth of field that can be provided by the objective optical system 7 is considerable. Therefore, a sufficient depth of field can be achieved for observation and diagnosis of an object without requiring any focusing operation. This simplifies the assembly of the capsule endoscope body 1. In addition, whereas plastic lenses previously would sometimes result in focusing failures due to the optical properties of plastic being more dependent on temperature and humidity, a lack of the need for focusing as in the present invention enables plastic lenses to be used. Therefore, improved yield as well as decreased cost of manufacture and assembly of the objective optical system within the capsule endoscope body should result.

The objective optical system of this embodiment has a wide field of view of approximately 140° that is obtained using a retro-focus-type lens arrangement that is formed of, in order from the object side, a lens group of negative refractive power that is formed of a negative lens element and a positive lens element, a brightness stop with a spatial frequency characteristic converter positioned at the brightness stop, and a lens group of positive refractive power that is formed of a positive lens element that is joined to a negative lens element. Because of the difficulty in orienting a capsule endoscope body within a patient, a wide-angle field of view objective optical system combined with the extended depth of field as available in the present invention is extremely useful in insuring that larger cavities within a living body are properly observed by the capsule endoscope. A sanded diffusing plate or a concave lens which results in a broadly diverging illumination beam can be placed in front of the light exit surface of the LED in order to supplement any shortage of light in the peripheral areas of the expanded field of view. In this way, an optical illumination system with a broad distribution of light is provided, and aids in ensuring that larger cavities within a living body are properly observed by the capsule endoscope. This embodiment includes, within the capsule endoscope body 1, an image processing unit 12, whose function is as discussed above.

It is useful for wireless capsule endoscopes, in order to save power, to use an image compression technique such as a JPEG format before transmitting the image data. The JPEG format affects the spatial frequency content of images that have been compressed using this format by omitting high spatial frequencies from the image data. The present embodiment's design enables image compression to occur with a fewer number of circuits, thus reducing the production cost. Furthermore, the spatial frequency property restoration and image compression are controlled for each capsule endoscope body, ensuring precise image reproduction without variations in the image quality due to production tolerances. Moreover, the spatial frequency property restoration is performed before the image compression, in order to minimize loss in image quality due to the JPEG format irreversibly omitting higher spatial frequencies. In this manner the quality of images obtained using the capsule endoscope is improved.

### Embodiment 2

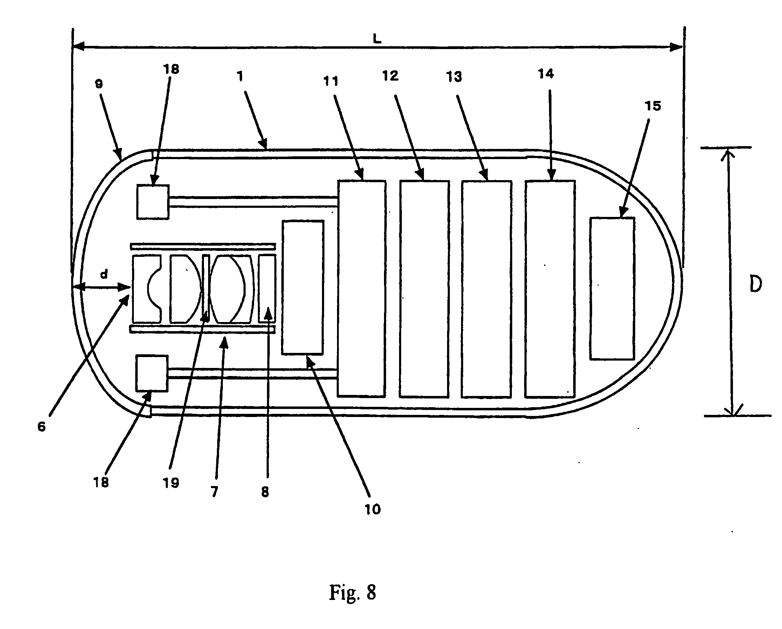

Fig. 8 shows the structure of the capsule endoscope body 1 of Embodiment 2, which is different from Embodiment 1 in that the tip portion of transparent cover 9 for covering the illumination and objective optical systems has a substantially oval shape. As described above, the depth of field of the imaging system of the capsule endoscope body is increased using the present invention. Therefore, even with a small distance d between the observation point and the first lens surface, focusing is achieved at the point that is in contact with the tip portion cover 9. Thus, the overall length L of the capsule body can be further reduced without degrading observations made with the capsule endoscope. This enables the tip portion of the cover to be formed of inexpensive, molded plastic having any shape. In this embodiment, an oval shape is used.

### Embodiment 3

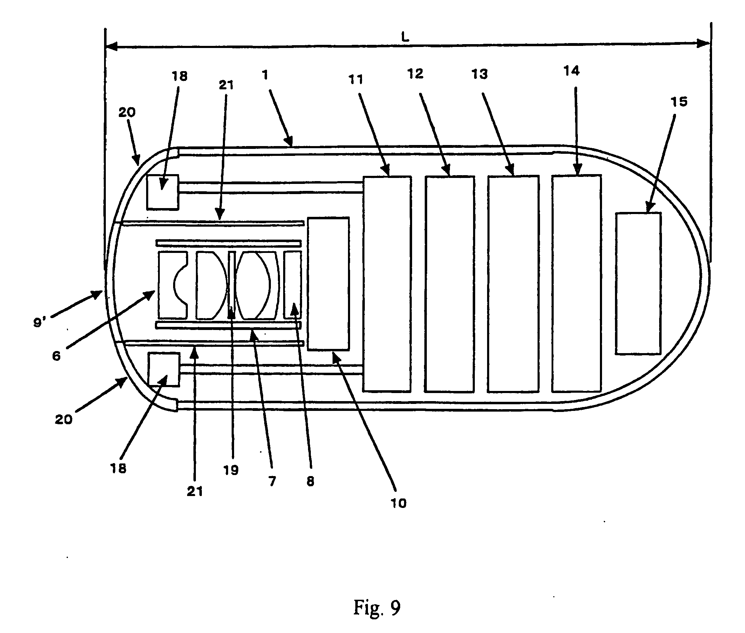

Fig. 9 shows the structure of the capsule endoscope body 1 of Embodiment 3, which is different from Embodiments 1 and 2 in the structure of the tip portion of transparent cover 9 and the presence of a shielding member 21 between the objective optical system 6 and the illumination system 18. The tip portion cover of this embodiment is formed of different materials for the part 9' that covers the objective optical system and for the part 20 that covers the illumination system.

The structure of Embodiment 1 wherein a single tip portion cover covers the objective optical system 7 and the illumination system 18 allows the light exiting from the optical illumination system 18 to reflect at the tip portion of the transparent cover 9, producing stray light, which then enters the objective optical system 7 and easily causes a flare in the field of view. To avoid this, a positional adjustment among the objective optical system, the illumination system 18, and the tip portion cover are performed for the stray light, if any, to prevent entry into the objective optical system. However, such an adjustment will make assembly of the capsule endoscope body 1 more difficult. The structure of this embodiment can easily prevent stray light from entering the objective optical system and, therefore, there is no need for a positional adjustment among the objective optical system 7, the illumination system 18, and tip portion cover. Therefore, the capsule endoscope body 1 is easier to assemble, enabling an increased yield to be achieved and reducing production cost.

### Embodiment 4

Embodiment 4 is different from Embodiment 1 in that it includes, in the receiver unit 16 of Fig. 5, a signal processing circuit 17 (for restoring the spatial frequency property so as to form images with an extended depth of field). Providing the signal processing circuit 17 in the receiver unit 16 instead of within the capsule endoscope body 1 can simplify the signal processing and circuit structure that is required within the capsule endoscope body 1. This allows further power savings and an extended time for observation and diagnosis of the capsule endoscope body within a patient, realizing a more practical capsule endoscope system. Furthermore, batteries having a smaller storage capacity and smaller volume can be used, allowing a further downsizing of the capsule. When irreversible compression such as JPEG format is used, the high spatial frequency components of an image are eliminated. Therefore, the receiver unit 16 receives image signals with reduced high spatial frequency content which varies according to the image compression ratio. The signal processing circuit 17, in the receiver unit 16, for restoring the spatial frequency property, can perform an optimized process that is targeted for the medium to low frequency property of the image signals. This can simplify the signal processing circuit 17, reducing the production cost of the capsule endoscope system. Generally, high frequency components for the image signals include noise from electrical elements such as the image sensors, and this noise can be amplified during image reproduction by the image processing circuit. This embodiment achieves a reproduced image with less noise by using signal processing that emphasizes the medium to low spatial frequency components and de-emphasizes the high spatial frequency components.

### Embodiment 5

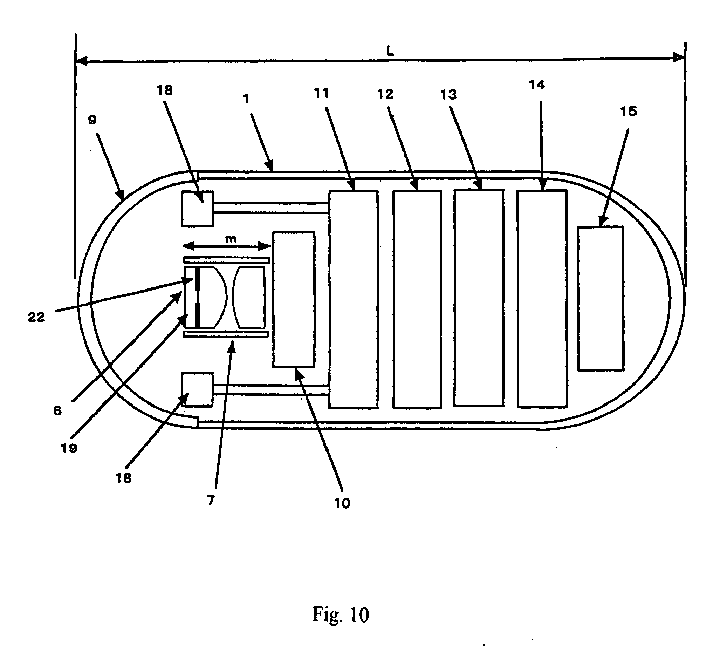

Fig. 10 shows the structure of the capsule endoscope body 1 of Embodiment 5, which is different from Embodiment 1 with regard to the structure of the objective optical system. The objective optical system of this embodiment includes a spatial frequency characteristic converter 19. The objective lens 6 is formed of two positive lens elements. Accordingly, the objective optical system is formed of, in order from the object side, the spatial frequency characteristic converter 19, a brightness stop 22, two plano-convex lenses, and the light receiving surface of a solid-state imaging element 10. Generally, an objective optical system formed of only positive lenses makes for a compact imaging unit but does not provide a sufficiently large back focus. The objective optical system 7 of this embodiment has the spatial frequency characteristic converter 19 very near the brightness stop 22, thereby reducing the overall length m of the objective optical system 7. This can further reduce the overall length L of the capsule endoscope body.

The objective optical system 7 of this embodiment does not include an infrared filter or color correction filter. As described in the prior art, conventional objective optical systems for endoscopes require an infrared filter or color correction filter. However, the capsule endoscope includes the illumination unit and imaging unit together within the capsule. Therefore, color reproduction for the imaging unit is determined according to the spectral intensity property of the illumination unit. For this reason, there is no need for a color correction filter in the objective optical system. In addition, a white LED is used as the illumination system. The white LED uses fluorescent substances to create desired colors and, therefore, does not produce significant amounts of ultraviolet or infrared light which may degrade observations using electronic image sensors.

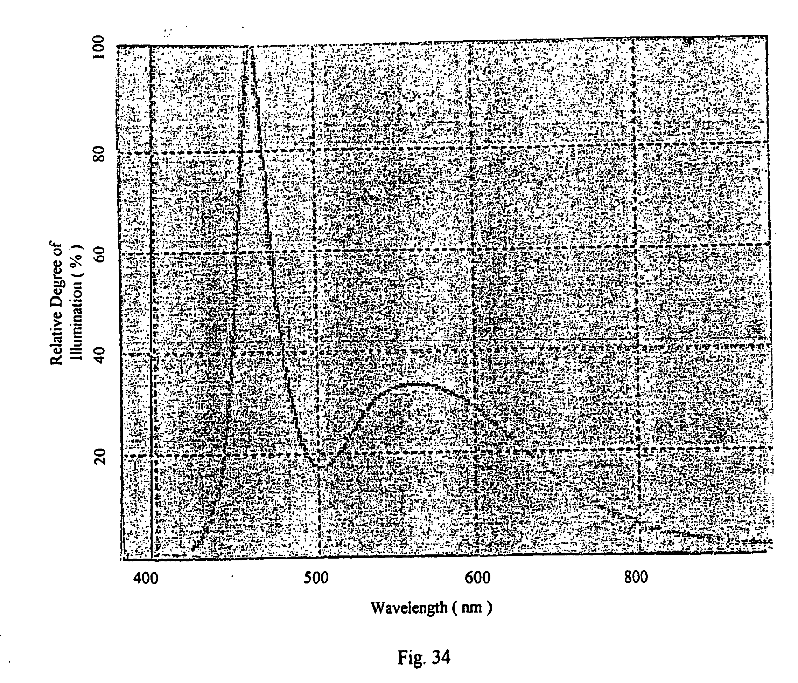

Fig. 34 shows the relative degree of spectral radiance (expressed as a percentage on the Y-axis) versus wavelength of emitted light (expressed in nm on the X-axis) of a white LED. As there are negligible ultraviolet wavelengths and almost no infrared wavelengths generated by the white LED, there is no need to include an infrared blocking filter or an ultraviolet blocking filter in the objective optical system. By eliminating the need for both infrared and color correction filters, a positive refractive power optical system that does not need a large back focus may be used.

### Embodiment 6

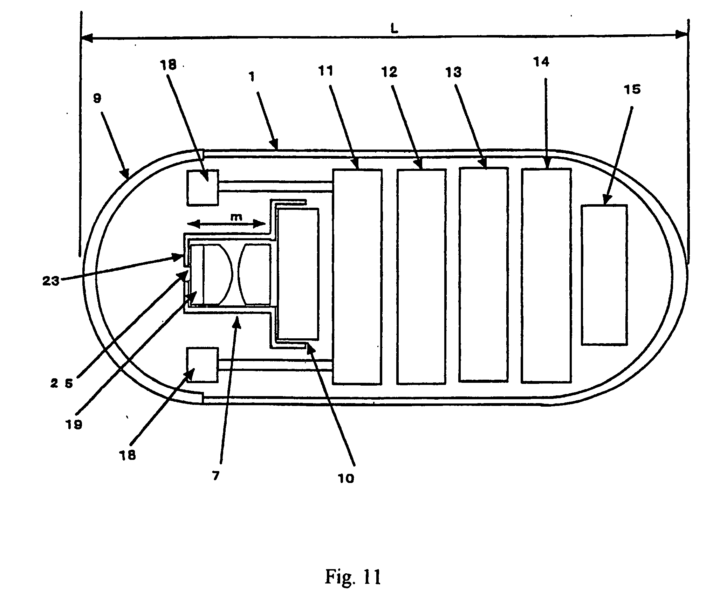

Fig. 11 shows the structure of the capsule endoscope body 1 of Embodiment 6, which is different from Embodiment 5 with regard to the structure of the objective optical system. The objective optical system 7 of this embodiment includes a spatial frequency characteristic converter 19. The objective lens system is formed of two positive lenses. Accordingly, the objective optical system is formed of, in order from the object side, a frame 23 that has an opening on the object side which serves as a brightness stop, the spatial frequency characteristic converter 19, two plano-convex lenses, and the light receiving surface of a solid- state imaging element 10. A frame 23 has an opening 25 on the object side that serves as a brightness stop, which is immediately followed by the spatial frequency characteristic converter 19. Positioning the spatial frequency characteristic converter 19 immediately after the brightness stop allows the objective optical system to be formed of only two positive lens elements.

The structure includes a capsule endoscope body that houses an illumination means 18, an objective optical unit 7, a solid-state imaging element 10, an image processing unit 12 for controlling the solid-state imaging element 10 and processing images, a total control unit 11, a wireless unit 13, an antenna 15, and a power unit 14, which are sealed within a capsule cover 1 and a transparent cover 9 as is shown in Fig. 11. Also, a receiver 16 is provided with a signal processing circuit 17 for reproducing the spatial frequency properties as shown in Fig. 5. The power unit 14 is a battery which supplies all the power necessary for the capsule endoscope. The solid-state imaging element 10 either a CCD or an MOS type image sensor.

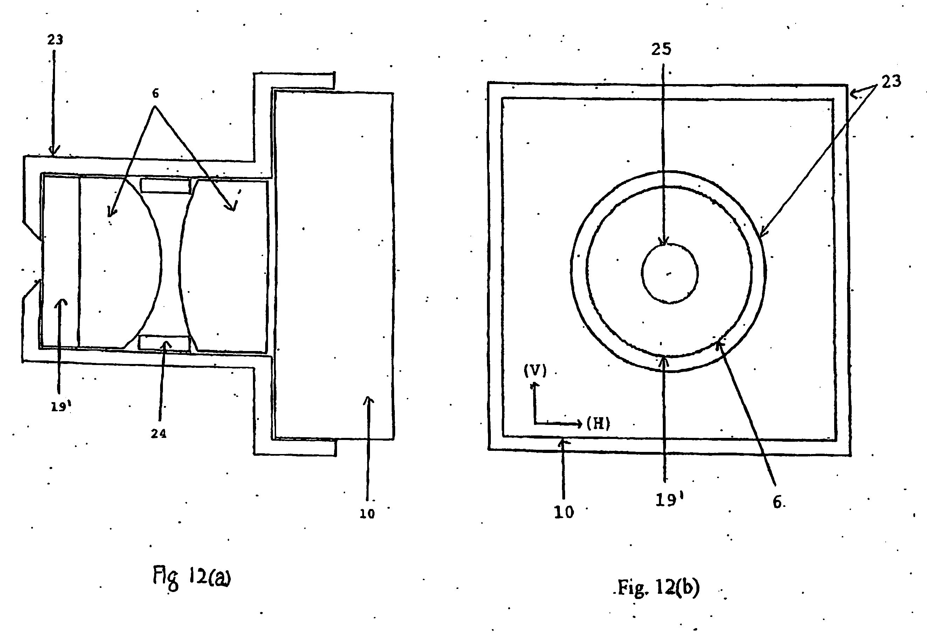

Figs. 12(a) and 12(b) show the detailed structure of the imaging unit. Fig. 12(a) is a side view and Fig. 12(b) is an end view as seen from the object side. Referring to Fig. 12(a) a pupil modulating element 19', a plano-convex lens 6, a ring 24 for spacing, a plano-convex lens 6, and a solid-state imaging element 10 are provided, in order from the object side, in a lens frame 23 having an opening 25. Referring to Fig. 12(b), the opening 25 of the lens frame 23 is a brightness stop and has a round shape. The pupil modulating element 19' has a circular periphery around the optical axis, as does the plano-convex lens 6. The pupil modulating element 19' has an outer diameter equal to that of the plano-convex lens 6.

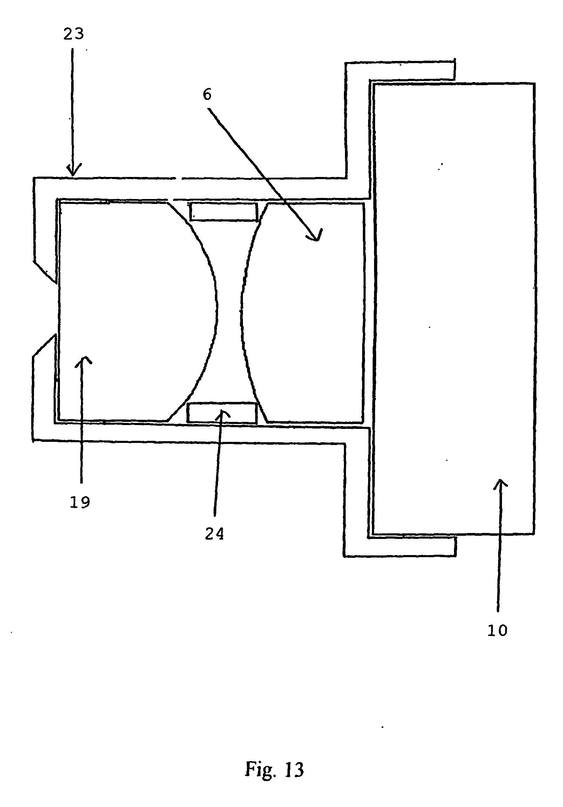

As is shown in Fig. 13, the number of parts is further reduced by combining the spatial frequency characteristic converter 19 and the plano-convex lens 6 into a single lens or bonding the oppositely oriented plano-convex lens 6 to the solid-state imaging element 10 in order to increase the assembly performance of the imaging unit. This is also useful to prevent the distances between parts that form the imaging unit from diverging so that the quality of the imaging is stable, while improving the yield.

### Embodiment 7

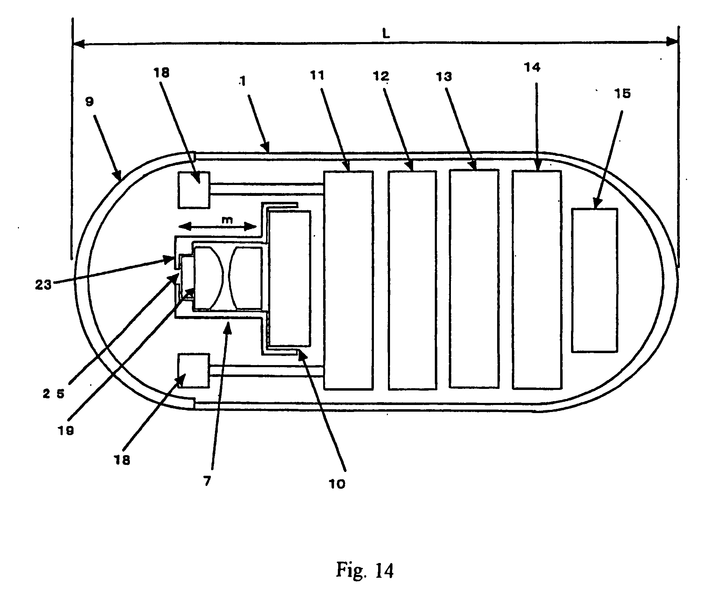

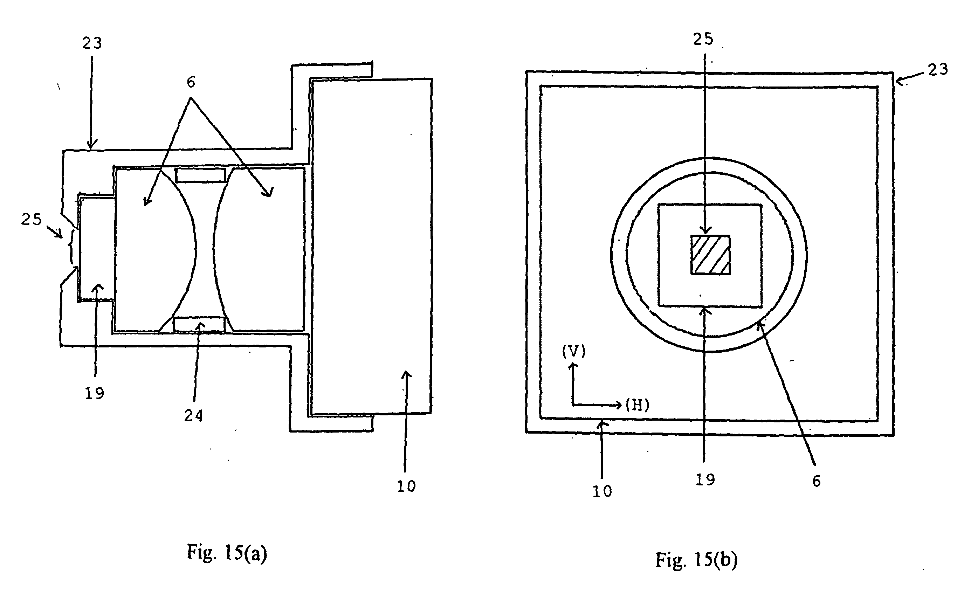

Fig. 14 shows the structure of the capsule endoscope body 1 of Embodiment 7, which is different from Embodiment 6 with regard to the structure of the objective optical system. Figs. 15(a) and 15(b) are detailed views of the imaging unit of Embodiment 7, with Fig. 15 (a) being a side view and Fig. 15(b) being an end view as seen from the object side. The objective optical system 7 includes a spatial frequency characteristic converter 19. The objective lens consists of two plano-convex lenses oriented with their convex sides facing one another, as indicated. Accordingly, the objective optical system consists of, in order from the object side, the spatial frequency characteristic converter 19, the two plano-convex lenses, and the light receiving surface of a solid-state imaging element 10. A frame 23 has an opening 25 on the object side that serves as a brightness stop, which is immediately followed by the spatial frequency characteristic converter 19.

Referring to Figs. 15(a) and 15(b), the brightness stop or opening 25 formed in the frame 23 is substantially a square. The spatial frequency characteristic converter 19 is also substantially a square. Accordingly, the frame 23 has a substantially square inner contour to house the spatial frequency characteristic converter 19. The solid-state imaging element 10 is also formed to be substantially square. Accordingly, the frame 23 has a substantially square inner contour to house the solid-state imaging element 10. A substantially square area of the spatial frequency characteristic converter 19 that faces the brightness stop or opening 25 has a free three-dimensional curved surface shown in Fig. 23. The vertical (V) and horizontal (H) directions of the pixel array of the solid-state imaging element 10 are aligned with the vertical and horizontal directions of the substantially square area of the spatial frequency characteristic converter 19 and the brightness stop or opening 25. This can maximize the spatial frequency property transforming performance in the imaging unit for the solid-state imaging element 10. This also contributes to optimization of the spatial frequency property transforming performance in the imaging unit, taking into account the vertical (V) and horizontal (H) resolutions of a monitor.

Using the frame structure of this embodiment eliminates the vertical and horizontal aligning operation of the solid-state imaging element 10, the spatial frequency characteristic converter 19, and the brightness stop or opening 25. Also, as with Embodiment 6, the parts forming the imaging unit can be inserted and fixed in the frame 23 for assembly. This facilitates assembly by dramatically reducing the labor and time required for assembly. The number of parts can be reduced by molding the spatial frequency characteristic converter 19 to the plano-convex lens that immediately follows it, further improving assembly performance.

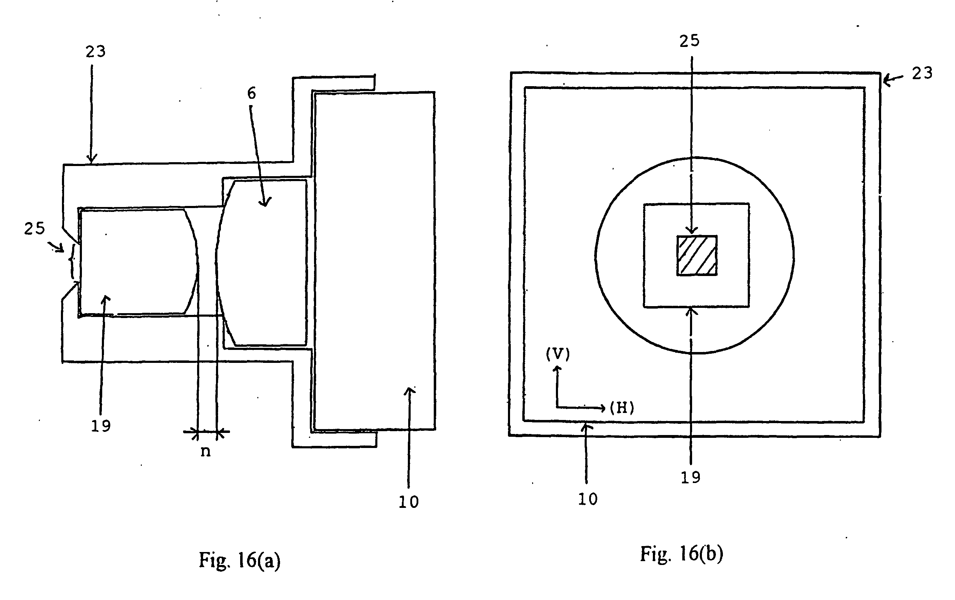

Figs. 16(a) and 16(b) show a modified embodiment of Embodiment 7, with Fig. 16(a) being a side view and Fig. 16(b) being an end view as seen from the object side. The imaging unit includes a spatial frequency characteristic converter and a plano-convex lens that are combined into a single lens 19 and another plano-convex lens 6, as illustrated. The frame 23 has a structure that determines the distance n between the spatial frequency characteristic converter 19 and the plano-convex lens 6 so that the clearance ring between the spatial frequency characteristic converter 19 and the plano-convex lens 6 is eliminated, thus reducing the number of parts. Also in this embodiment, the plano-convex lens 6 and the solid-state imaging element 10 can be bonded together to reduce the number of parts, further improving assembly performance of the imaging unit.

### Embodiment 8

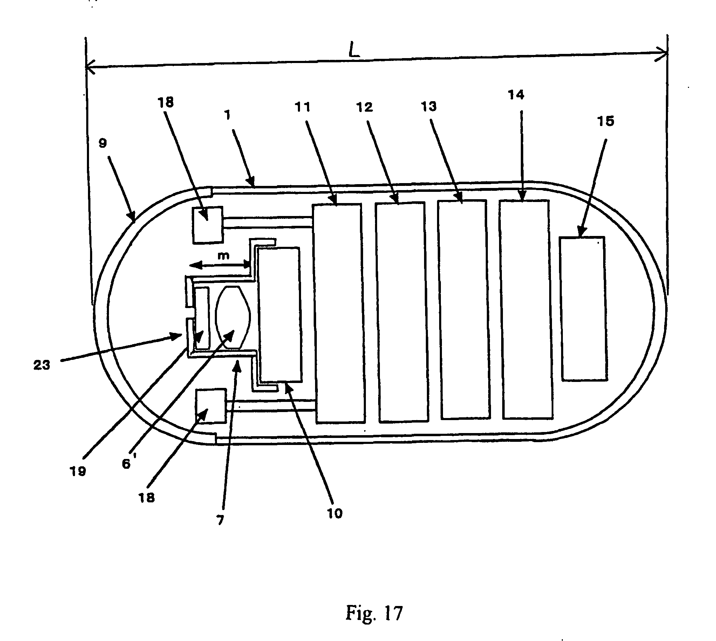

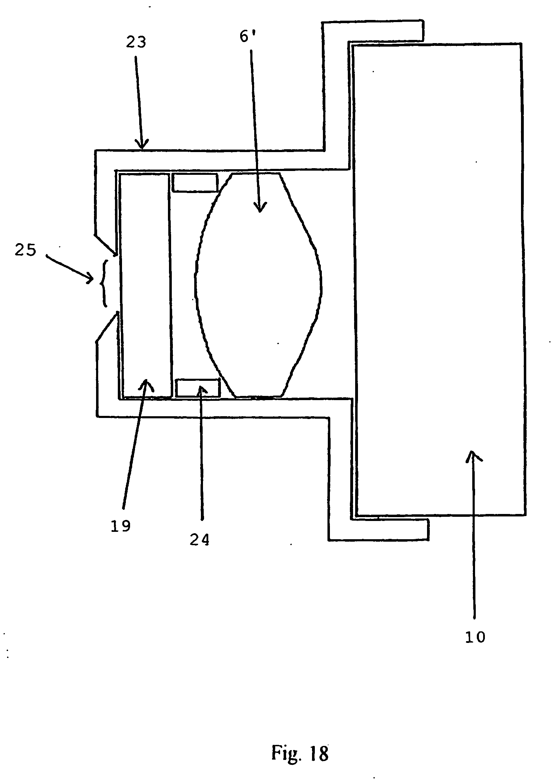

Fig. 17 shows the structure of the capsule endoscope body of Embodiment 8, which is different from that of Embodiments 5, 6, and 7 in the structure of the objective optical system. Fig. 18 is a detailed view of the imaging unit of Embodiment 8. The objective optical system includes a spatial frequency characteristic converter 19, as illustrated element. The objective lens consists of a single, aspherical biconvex lens 6'. Accordingly, the imaging unit consists of, in the following order from the object side, the spatial frequency characteristic converter 19, the aspherical biconvex lens 6', and the light receiving surface of a solid-state imaging element 10. A frame 23 has an opening 25 on the object side that serves as a brightness stop, which is immediately followed by the spatial frequency characteristic converter 19.

The structure consisting of a single aspherical biconvex lens can not have a sufficiently large back focus. Therefore, the spatial frequency characteristic converter 19 is positioned at the position of the brightness stop of the objective optical system. An aspherical lens is capable of correcting the field curvature and spherical aberration on its own. This can result in an extremely compact imaging unit with a large depth of field and satisfactorily corrected aberrations while having a wide field angle. Using this imaging unit contributes to the further reduction in the overall length L of the capsule endoscope body. Also, as with Embodiment 6, the parts forming the imaging unit can be inserted and fixed in the frame 23, in order, for assembly. In other words, the spatial frequency characteristic converter 19, clearance ring 24, aspherical biconvex lens 6, and solid-state imaging element 10 are inserted and fixed in the frame 23 in order from the object side. This reduces the cost of assembly of the imaging unit.

### Embodiment 9

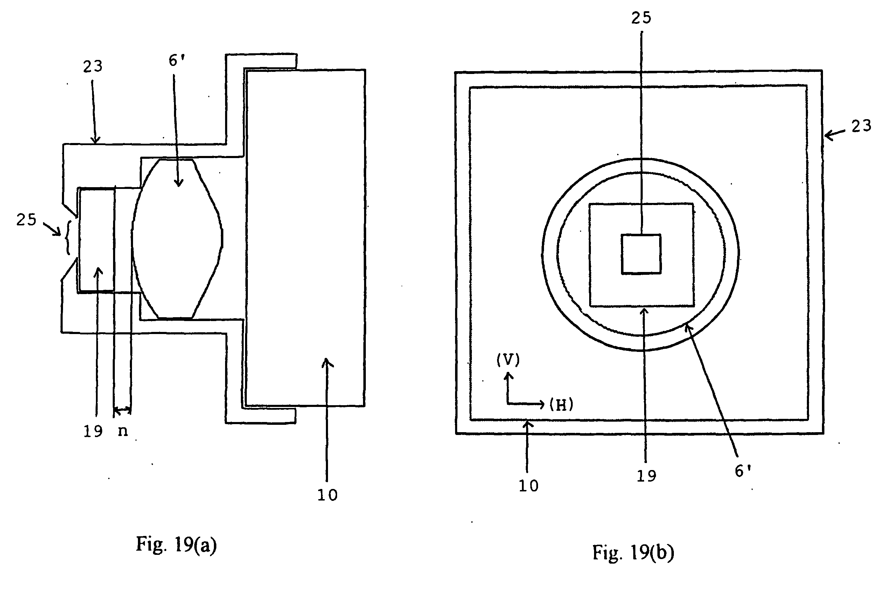

Figs. 19(a) and 19(b) are detailed views of the imaging unit of Embodiment 9, with Fig. 19(a) being a side view and Fig. 19(b) being an end view, as seen from the object side. Embodiment 9 is different from Embodiment 8 with regard to the structure of the objective optical system. The objective optical system includes a spatial frequency characteristic converter 19. The objective lens consists of a single aspherical lens of positive refractive power. Accordingly, the imaging unit is formed of, in order from the object side, a spatial frequency characteristic converter 19, an aspherical biconvex lens 6', and the light receiving surface of a solid-state imaging element 10. A frame 23 has an opening 25 on the object side that serves as a brightness stop, which is immediately followed by a spatial frequency characteristic converter 19. The brightness stop or opening 25 formed in the frame 23 is substantially a square. The spatial frequency characteristic converter 19 is also formed so as to be substantially square. Accordingly, the frame 23 has a substantially square-shaped inner contour so as to house the spatial frequency characteristic converter 19.

The solid-state imaging element 10 is formed to be substantially square. Accordingly, the frame 23 has a substantially square-shaped inner contour in order to house the solid-state imaging element 10. The substantially square-shaped area of the spatial frequency characteristic converter 19 that faces the brightness stop or opening 25 has a free three-dimensional curved surface shown in Fig. 23. The vertical (V) and horizontal (H) directions of the pixel array of the solid-state imaging element 10 are aligned with the vertical and horizontal directions of the substantially square-shaped area of the spatial frequency characteristic converter 19 and the brightness stop or opening 25. This can enhance the imaging performance of the solid-state imaging element 10.

The frame 23 has a structure that determines the distance n between the spatial frequency characteristic converter 19 and the aspherical biconvex lens 6'. This eliminates the need for a clearance ring between the spatial frequency characteristic converter 19 and the aspherical biconvex lens 6', further reducing the number of parts. Using the frame structure of this embodiment eliminates the vertical and horizontal aligning operation as in Embodiment 8, namely, of the solid-state imaging element 10, the spatial frequency characteristic converter 19, and the brightness stop or opening 25. As with Embodiment 8, the parts forming the imaging unit can be inserted and fixed easily into the frame 23 during the assembly process.

### Embodiment 10

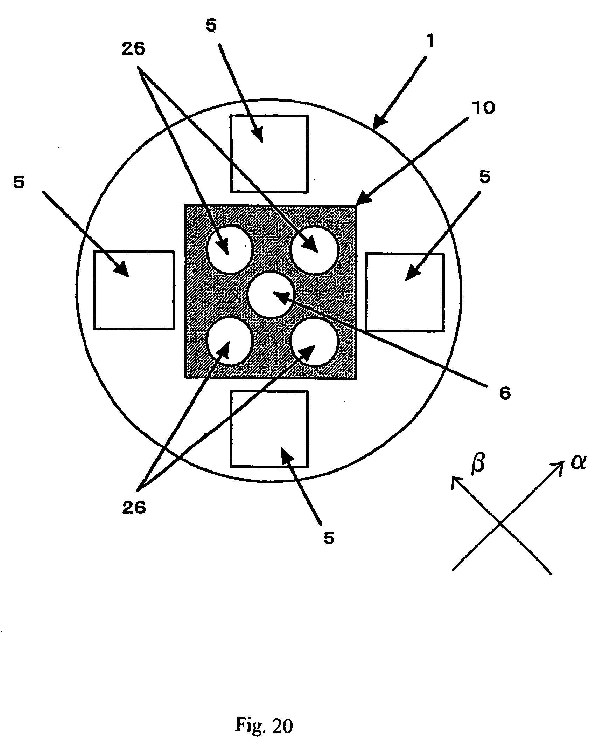

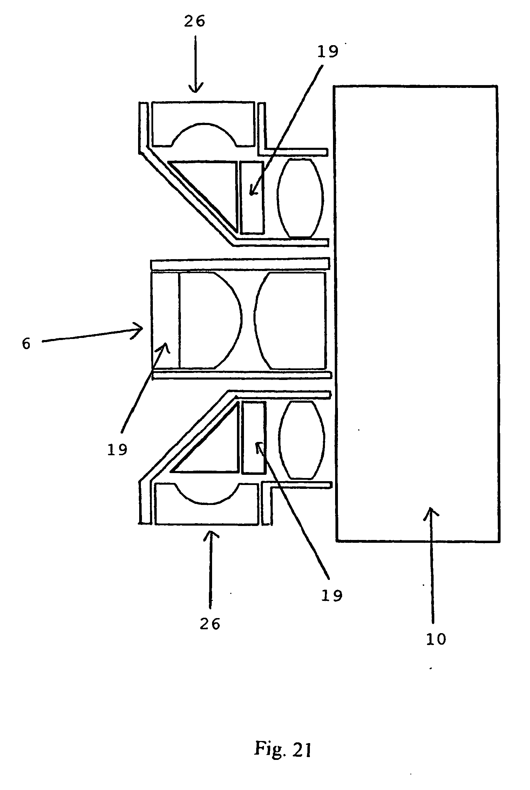

Fig. 20 is a front view as seen from the object side of the capsule endoscope body 1 of Embodiment 10. Fig. 21 is a sectional view of the imaging unit in the "α" direction indicated in Fig. 20. This embodiment uses plural objective optical systems 26, a central objective lens 6, and a single solid-state imaging element 10. An objective optical lens 6 for observation in a direct viewing direction and objective optical systems 26 for observation in a perspective viewing direction are provided. Each objective optical system has a spatial frequency characteristic converter 19.

Generally, it is difficult to control the observation direction of a capsule endoscope body within a patient. Therefore, extending the viewing range of the imaging system is necessary to provide maximum observation and diagnosis within the body. The present embodiment uses plural objective optical systems that have different viewing directions. This allows an extended range of observation within the body. Each objective optical system is provided with a spatial frequency characteristic converter 19. Therefore, the focusing operation is eliminated even though plural objective optical systems are used. As a result, the capsule endoscope body of this embodiment allows an extended range of observation and is easy to assemble.

The image processing unit processes images which are observed from several different directions and are formed on the solid-state imaging element 10 by the plural objective optical system so as to create a single, wide angle image with little distortion. Parallax that occurs when one and the same object is observed by respective objective optical systems can be used to create a three-dimensional image. A capsule endoscope body that combines an imaging unit that includes plural objective optical systems which have different viewing directions with an image processing unit as discussed above, can achieve images that are optimized for the particular observation and diagnosis conditions within the capsule endoscope body. This embodiment uses five objective optical systems, but other numbers of objective optical systems may be used as well.

The fact that the capsule endoscope can be continuously powered from an external source solves the problem of a shortage of power within capsule endoscope bodies. This allows an extended observation time for diagnosis within the body. Furthermore, all of the objective optical systems of Embodiments 1 to 10 can use plastic lenses. As described above, the imaging system of the present invention can solve the problem that the prior art objective optical systems of capsule endoscopes suffer from, namely deteriorated imaging performance and failure to focus because of the temperature and moisture variations in the body. Using plastic lenses with a spatial frequency characteristic converter 19 leads to a reduction in the weight of the optical systems, achieving a light-weight capsule endoscope. This facilitates location control by magnetic induction of the capsule endoscope body within a patient. Plastic lenses also contribute significantly to reducing production costs, and the reduced production costs enable the capsule endoscope body to be discarded after a single use.

### Embodiment 11

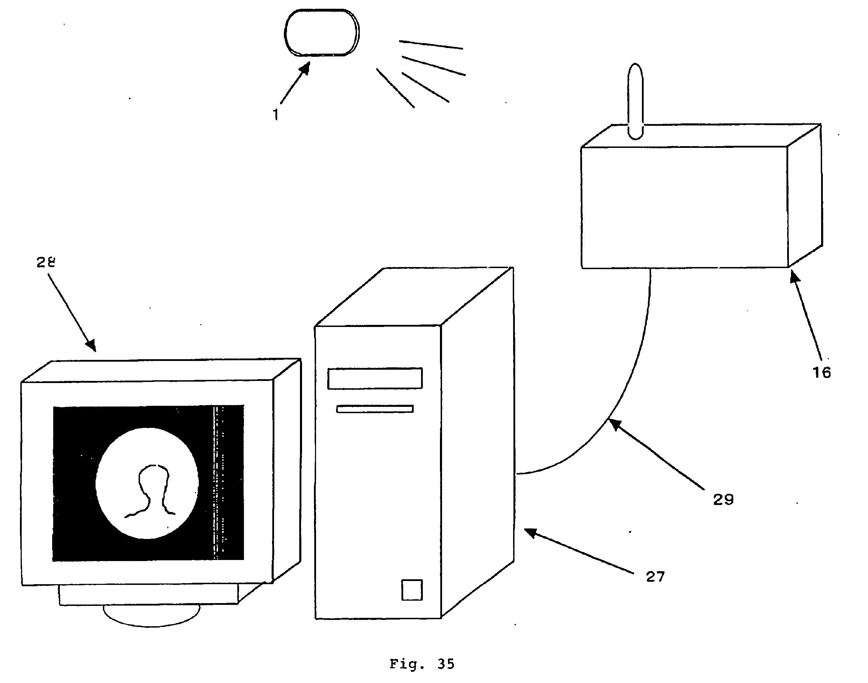

Fig. 35 shows the eleventh embodiment of this invention. This embodiment is different from the embodiment 4 in a point that the image data processing apparatus 27 includes a signal processing circuit 17 which restores the spatial frequency property so as to form images with an extended depth of field. Reference numeral 28 denotes a monitoring TV for displaying an image of an object captured by the capsule endoscope.

This enables to simplify the electronic circuit of the receiver 16 and to reduce power requirements of the receiver 16, that is to say, to reduce the size of battery in the receiver 16. Therefore, the receiver 16 is miniaturized and this serves to reduce the burden of the patient wearing the receiver while he or she is under examination.

A personal computer can be used as the image data processing apparatus 27 that includes the signal processing circuit 16. In this case, version up or update of the software used for restoring is easily done by using CD-ROM or the like. In addition, by exchanging the computer into a new one having a high speed CPU or a portable computer that can be easily carried by a doctor. This enables to set up a flexible capsule endoscope observation system which satisfies various demands of the doctors.

In this figure, the receiver 17 and the image data processing apparatus are electrically connected with a cable 29 for transmitting an image data signal. However, a wireless transmission ca be used for the same purpose. Small data storing medium can be used to transfer the image data from the receiver 17 to the image data processing apparatus.

The invention being thus described, it will be obvious that the same may be varied in many ways. For example, each of the power units 14 of Embodiments 1 to 10 of the present invention can at least in part be powered by an external power source, with the energy being carried to the capsule endoscope body via electromagnetic waves, such as microwaves. An antenna 15 for receiving microwaves may be provided, as well as a power unit 14 which transforms the microwaves into electrical energy which is then stored in a known device, such as a capacitor or rechargeable battery. The capsule can be further downsized by sharing the antenna 15 with other functions such as image transmission. In this way electric energy can be continuously supplied to the capsule and the energy storage device can be made smaller, which enables the capsule body to be made smaller. Further, the objective optical system can include glass lenses so long as they do not include harmful substances such as arsenic and lead, as these substances would be unsafe for use within a human body and may pose disposal problems.

## Claims

1. A capsule endoscope body (1) comprising the following components that are sealed within the capsule endoscope body (1), an illumination system (18) for illuminating an interior part of a hollow cavity, an imaging system for imaging the interior part that is illuminated by the illumination system (18), said imaging system including an objective optical system (7) and an image sensor (10) which scans the images so as to convert the images into electrical output signals, and a transmitter (13, 15) for transmitting the signals output by the image sensor (10), **characterized in that**
the imaging system further including a spatial frequency characteristic converter (19) which causes the optical transfer function of the imaging system to remain essentially constant within some range of in-focus position and
within the capsule endoscope body (1) there is further sealed a signal processing circuit for performing spatial frequency restoration prior to image compression.

2. The capsule endoscope body according to Claim 1, wherein the objective optical system (7) consists of a single aspherical lens of positive refractive power.

3. The capsule endoscope body according to Claim 1, wherein the objective optical system (7) is formed of only two lens groups, each of positive refractive power.

4. The capsule endoscope body according to Claim 1, wherein the objective optical system (7) is formed of, in order from the object side, a first lens group having an overall negative refractive power, and a second lens group having an overall positive refractive power.

5. The capsule endoscope body according to Claim 1, wherein the spatial frequency characteristic converter (19) has an aperture which is shaped the same as the light receiving part of the image sensor (10).

6. The capsule endoscope body according to Claim 1, wherein the image sensor (10) is a MOS-type image sensor or a CCD image sensor.

7. The capsule endoscope body according to Claim 1, wherein power for operating the capsule endoscope body (1) is provided by at least one battery (14) located within the sealed capsule endoscope body (1).

8. The capsule endoscope body according to Claim 1, wherein power for operating the capsule endoscope body (1) is provided at least in part by the capsule endoscope body (1) receiving electromagnetic energy from an energy source which is located outside the sealed capsule endoscope body (1).

9. The capsule endoscope body according to Claim 1, wherein the objective optical system (7) is formed of plastic lenses.

10. The capsule endoscope body according to Claim 1, and further including within said capsule endoscope body (1) a signal processing circuit for adjusting for manufacturing variations of the imaging system.

11. The capsule endoscope body according to Claim 1, wherein the sealed capsule has a transparent, substantially oval-shaped, tip portion cover (9) that covers the front of the objective optical system (7) and the illumination system (18).

12. The capsule endoscope body according to Claim 1, wherein the sealed capsule has a transparent tip portion cover (9) that covers the front of the objective optical system (7) and the illumination system (18), with the shape of the tip portion cover (9) at a part that covers the front of the objective optical system (7) being different from the shape of the tip portion cover (9) at a part that covers the front of the illumination system (18).

13. The capsule endoscope body according to Claim 1, wherein the spatial frequency characteristic converter (19) is positioned substantially at a pupil of the objective optical system (7).

14. The capsule endoscope body according to Claim 1, 2 or 3, wherein a frame (23) for housing the objective optical system (7) has an opening on the object side of the objective optical system (7) which serves as a brightness stop, and the spatial frequency characteristic converter (19) is positioned substantially at the brightness stop.

15. The capsule endoscope body according to Claim 1, wherein said spatial frequency characteristic converter (19) has a circular aperture.

16. The capsule endoscope body according to Claim 1, wherein the imaging system further includes an additional objective optical system.

17. A capsule endoscope system comprising a capsule endoscope body (1) defined in claim 1 and a capsule endoscope receiver (16) for receiving image data transmitted from the capsule endoscope body including a signal processor (17) for restoring the spatial frequency content of the modified image data.

18. A capsule endoscope system comprising a capsule endoscope body (1) defined in claim 10 and a capsule endoscope receiver (16) for receiving image data transmitted from the capsule endoscope body (1).

19. A system comprising a capsule endoscope body (1) according to claim 1, a capsule endoscope receiver (16) for receiving image data transmitted from the capsule endoscope body (1), and an image data processing apparatus (27) for processing the image data and displaying the image on a monitor (28), the image data processing apparatus (27) including a signal processor (17) for restoring the spatial frequency content of the modified image data.

## Patentansprüche

1. Kapselendoskopkörper (1) aufweisend die folgenden in dem Kapselendoskopkörper (1) eingeschlossenen Komponenten:
ein Beleuchtungssystem (18) zum Beleuchten eines inneren Bereichs eines Hohlraums,
ein bildgebendes System zum Abbilden des durch das Beleuchtungssystem (18) beleuchteten inneren Bereichs, wobei das bildgebende System ein Objektiv-optisches System (7) und einen die Bilder abtastenden Bildsensor (10) zum Wandeln der Bilder in elektrische Ausgangssignale enthält, und
einen Sender (13, 15) zum Senden der von dem Bildsensor (10) ausgegebenen Signale, **dadurch gekennzeichnet, dass**
das bildgebende System ferner einen Raumfrequenzcharakteristikwandler (19) enthält, der bewirkt, dass die optische Übertragungsfunktion des bildgebenden Systems innerhalb eines Bereichs einer Scharfstellung im Wesentlichen konstant bleibt, wobei
in dem Kapselendoskopköper (1) ferner eine Signalverarbeitungsschaltung zum Ausführen von Raumfrequenzwiederherstellung vor einer Bildkomprimierung eingeschlossen ist.

2. Kapselendoskopkörper nach Anspruch 1, bei dem das Objektiv-optische System (7) aus einer einzelnen asphärischen Linse mit positiver Brechkraft besteht.

3. Kapselendoskopkörper nach Anspruch 1, bei dem das Objektiv-optische System (7) nur aus zwei Linsengruppen gebildet ist, von denen jede eine positive Brechkraft aufweist.

4. Kapselendoskopkörper nach Anspruch 1, bei dem das Objektiv-optische System (7), in der Reihenfolge von der Objektseite, gebildet ist aus einer ersten Linsengruppe mit einer insgesamt negativen Brechkraft und einer zweiten Linsengruppe mit einer insgesamt positiven Brechkraft.

5. Kapselendoskopkörper nach Anspruch 1, bei dem der Raumfrequenzcharakteristikwandler (19) eine Öffnung aufweist, die genauso wie das Lichtaufnahmeteil des Bildsensors (10) geformt ist.

6. Kapselendoskopkörper nach Anspruch 1, bei dem der Bildsensor (10) ein MOS-Typ-Bildsensor oder ein CCD-Bildsensor ist.

7. Kapselendoskopkörper nach Anspruch 1, bei dem Energie zum Betreiben des Kapselendoskopkörpers (1) durch mindestens eine Batterie (14) bereitgestellt wird, die in dem verschlossenen Kapselendoskopkörper (1) angeordnet ist.

8. Kapselendoskopkörper nach Anspruch 1, bei dem Energie zum Betreiben des Kapselendoskopkörpers (1) zumindest teilweise durch den Kapselendoskopkörper (1) bereitgestellt wird, der elektromagnetische Energie von einer außerhalb des abgeschlossenen Kapselendoskopkörpers (1) angeordneten Energiequelle erhält.

9. Kapselendoskopkörper nach Anspruch 1, bei dem das Objektiv-optische System (7) aus Kunststofflinsen gebildet ist.

10. Kapselendoskopkörper nach Anspruch 1, welches ferner innerhalb des Kapselendoskopkörpers (1) eine Signalverarbeitungsschaltung zum Ausgleichen von Herstellungsabweichungen des bildgebenden Systems enthält.

11. Kapselendoskopkörper nach Anspruch 1, bei dem die abgeschlossene Kapsel eine transparente, im Wesentlichen ovalförmige Vorderteilabdeckung (9) aufweist, welche die Vorderseite des Objektiv-optischen Systems (7) und des bildgebenden Systems (18) abdeckt.

12. Kapselendoskopkörper nach Anspruch 1, bei dem die abgeschlossene Kapsel eine transparente Vorderteilabdeckung (9) aufweist, welche die Vorderseite des Objektiv-optischen Systems (7) und des bildgebenden Systems (18) abdeckt, wobei sich die Form der Vorderteilabdeckung (9) in einem Bereich, der die Vorderseite des Objekt-optischen Systems (7) abdeckt, von der Form der Vorderteilabdeckung (9) in einem Bereich, der die Vorderseite des bildgebenden Systems (18) abdeckt, unterscheidet.

13. Kapselendoskopkörper nach Anspruch 1, bei dem der Raumfrequenzcharakteristikwandler (19) im Wesentlichen bei einer Pupille des Objektiv-optischen Systems (7) angeordnet ist.

14. Kapselendoskopkörper nach Anspruch 1, 2 oder 3, bei dem ein Rahmen (23) zum Aufnehmen des Objektiv-optischen Systems (7) eine Öffnung an der Objektseite des Objekt-optischen Systems (7) aufweist, die als Helligkeitsblende dient, wobei der Raumfrequenzcharakteristikwandler (19) im Wesentlichen bei der Helligkeitsblende angeordnet ist.

15. Kapselendoskopkörper nach Anspruch 1, bei dem der Raumfrequenzcharakteristikwandler (19) eine kreisförmige Öffnung aufweist.

16. Kapselendoskopkörper nach Anspruch 1, bei dem das bildgebende System ferner ein zusätzliches Objektiv-optisches System enthält.

17. Kapselendoskopsystem aufweisend einen Kapselendoskopkörper (1) gemäß Anspruch 1 und einen Kapselendoskopempfänger (16) zum Empfangen von von dem Kapselendoskopkörper gesendeten Bilddaten, enthaltend einen Signalprozessor (17) zum Wiederherstellen des Raumfrequenzinhalts der geänderten Bilddaten.

18. Kapselendoskopsystem aufweisend einen Kapselendoskopkörper (1) gemäß Anspruch 10 und einen Kapselendoskopempfänger (16) zum Empfangen von von dem Kapselendoskopkörper (1) gesendeten Bilddaten.

19. System aufweisend einen Kapselendoskopkörper (1) gemäß Anspruch 1, einen Kapselendoskopempfänger (16) zum Empfangen von von dem Kapselendoskopkörper (1) gesendeten Bilddaten und eine Bilddatenverarbeitungsvorrichtung (27) zum Verarbeiten der Bilddaten und Anzeigen des Bildes auf einem Monitor (28), wobei die Bilddatenverarbeitungsvorrichtung (27) einen Signalprozessor (17) zum Wiederherstellen des Raumfrequenzinhalts der geänderten Bilddaten enthält.

## Revendications

1. Corps d'endoscope à capsule (1) comprenant les composants suivants qui sont scellés à l'intérieur du corps d'endoscope à capsule (1) : un système d'éclairage (18) pour éclairer une partie intérieure d'une cavité creuse, un système d'imagerie pour imager la partie intérieure qui est éclairée par le système d'éclairage (18), ledit système d'imagerie incluant un système optique d'objectif (7) et un capteur d'images (10) qui balaye les images de façon à convertir les images en signaux de sortie électriques, et un transmetteur (13, 15) pour transmettre les signaux délivrés en sortie par le capteur d'images (10), **caractérisé en ce que**
le système d'imagerie incluant en outre un convertisseur de caractéristique de fréquence spatiale (19) qui fait que la fonction de transfert optique du système d'imagerie reste essentiellement constante à l'intérieur d'une certaine plage de position de focalisation et
à l'intérieur du corps d'endoscope à capsule (1), il est en outre scellé un circuit de traitement de signaux pour effectuer une restauration de fréquence spatiale avant une compression d'image.

2. Corps d'endoscope à capsule selon la revendication 1, dans lequel le système optique d'objectif (7) consiste en une lentille asphérique unique à pouvoir de réfraction positive.

3. Corps d'endoscope à capsule selon la revendication 1, dans lequel le système optique d'objectif (7) est formé de seulement deux groupes de lentilles, chacun à pouvoir de réfraction positive.

4. Corps d'endoscope à capsule selon la revendication 1, dans lequel le système optique d'objectif (7) est formé de, dans cet ordre à partir du côté objet, un premier groupe de lentilles ayant un pouvoir de réfraction générale négative, et un deuxième groupe de lentilles ayant un pouvoir de réfraction générale positive.

5. Corps d'endoscope à capsule selon la revendication 1, dans lequel le convertisseur de caractéristique de fréquence spatiale (19) a une ouverture qui a la même forme que la partie de réception de lumière du capteur d'images (10).

6. Corps d'endoscope à capsule selon la revendication 1, dans lequel le capteur d'images (10) est un capteur d'images de type MOS ou un capteur d'images CCD.

7. Corps d'endoscope à capsule selon la revendication 1, dans lequel l'énergie pour faire fonctionner le corps d'endoscope à capsule (1) est apportée par au moins une batterie (14) située à l'intérieur du corps d'endoscope à capsule (1) scellé.

8. Corps d'endoscope à capsule selon la revendication 1, dans lequel l'énergie pour faire fonctionner le corps d'endoscope à capsule (1) est apportée au moins en partie par le corps d'endoscope à capsule (1) recevant une énergie électromagnétique provenant d'une source d'énergie qui est située à l'extérieur du corps d'endoscope à capsule (1) scellé.

9. Corps d'endoscope à capsule selon la revendication 1, dans lequel le système optique d'objectif (7) est formé de lentilles en plastique.

10. Corps d'endoscope à capsule selon la revendication 1, et incluant en outre à l'intérieur dudit corps d'endoscope à capsule (1) un circuit de traitement de signaux pour ajuster en fonction de variations de fabrication du système d'imagerie.

11. Corps d'endoscope à capsule selon la revendication 1, dans lequel la capsule scellée a un recouvrement de partie de pointe (9) transparent de forme substantiellement ovale qui recouvre l'avant du système optique d'objectif (7) et du système d'éclairage (18).

12. Corps d'endoscope à capsule selon la revendication 1, dans lequel la capsule scellée a un recouvrement de partie de pointe (9) transparent qui recouvre l'avant du système optique d'objectif (7) et du système d'éclairage (18), avec la forme du recouvrement de partie de pointe (9) au niveau d'une partie qui recouvre l'avant du système optique d'objectif (7) étant différente de la forme du recouvrement de partie de pointe (9) au niveau d'une partie qui recouvre l'avant du système d'éclairage (18).

13. Corps d'endoscope à capsule selon la revendication 1, dans lequel le convertisseur de caractéristique de fréquence spatiale (19) est positionné substantiellement au niveau d'une pupille du système optique d'objectif (7).

14. Corps d'endoscope à capsule selon la revendication 1, 2 ou 3, dans lequel un cadre (23) pour recevoir le système optique d'objectif (7) a une ouverture sur le côté objet du système optique d'objectif (7) qui sert de diaphragme de luminosité, et le convertisseur de caractéristique de fréquence spatiale (19) est positionné substantiellement au niveau du diaphragme de luminosité.

15. Corps d'endoscope à capsule selon la revendication 1, dans lequel ledit convertisseur de caractéristique de fréquence spatiale (19) a une ouverture circulaire.

16. Corps d'endoscope à capsule selon la revendication 1, dans lequel le système d'imagerie inclut en outre un système optique d'objectif additionnel.

17. Système d'endoscope à capsule comprenant un corps d'endoscope à capsule (1) défini dans la revendication 1 et un récepteur d'endoscope à capsule (16) pour recevoir des données d'image transmises du corps d'endoscope à capsule incluant un processeur de signaux (17) pour restaurer le contenu en fréquence spatiale des données d'image modifiées.

18. Système d'endoscope à capsule comprenant un corps d'endoscope à capsule (1) défini dans la revendication 10 et un récepteur d'endoscope à capsule (16) pour recevoir des données d'image transmises du corps d'endoscope à capsule (1).

19. Système comprenant un corps d'endoscope à capsule (1) selon la revendication 1, un récepteur d'endoscope à capsule (16) pour recevoir des données d'image transmises du corps d'endoscope à capsule (1) et un appareil de traitement de données d'image (27) pour traiter les données d'image et afficher l'image sur un moniteur (28), l'appareil de traitement de données d'image (27) incluant un processeur de signaux (17) pour restaurer le contenu en fréquence spatiale des données d'image modifiées.
